(19) Europäisches Patentamt
European Patent Office
Office européen des brevets

(11) EP 2 131 841 B1

(12) **EUROPEAN PATENT SPECIFICATION**

(45) Date of publication and mention of the grant of the patent:
**01.08.2012 Bulletin 2012/31**

(21) Application number: **08724934.8**

(22) Date of filing: **29.01.2008**

(51) Int Cl.:
***A61K 31/437*** *(2006.01)* ***A61K 31/485*** *(2006.01)*
***A61P 25/04*** *(2006.01)*

(86) International application number:
**PCT/US2008/001187**

(87) International publication number:
**WO 2008/094571 (07.08.2008 Gazette 2008/32)**

(54) **METHODS FOR TREATING ACUTE PAIN**

VERFAHREN ZUR BEHANDLUNG VON AKUTEN SCHMERZEN

PROCÉDÉS DE TRAITEMENT DE LA DOULEUR AIGUË

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.01.2007 US 898362 P**

(43) Date of publication of application:
**16.12.2009 Bulletin 2009/51**

(73) Proprietors:
- **Avigen, Inc.**
  **Alameda, CA 94502-6541 (US)**
- **The Regents of The University of Colorado, A Body Corporate**
  **Boulder, CO 80309-0588 (US)**

(72) Inventors:
- **JOHNSON, Kirk, W.**
  **Alameda, CA 94502 (US)**
- **WATKINS, Linda**
  **Boulder, CO 80309-0345 (US)**
- **HUTCHINSON, Mark**
  **Adelaide, South Australia 5005 (AU)**

(74) Representative: **HOFFMANN EITLE**
**Patent- und Rechtsanwälte**
**Arabellastraße 4**
**81925 München (DE)**

(56) References cited:
**WO-A-2006/063048 WO-A-2007/146087**

- **WATKINS ET AL: "Glia: novel counter-regulators of opioid analgesia" TRENDS IN NEUROSCIENCE, ELSEVIER, AMSTERDAM, NL, vol. 28, no. 12, 1 December 2005 (2005-12-01), pages 661-669, XP005171903 ISSN: 0166-2236**
- **JOHNSON ET AL: "72 AV411: A UNIQUE, ORALLY ACTIVE GLIAL INHIBITOR FOR NEUROPATHIC PAIN" EUROPEAN JOURNAL OF PAIN, SAUNDERS, LONDON, GB, vol. 10, 1 September 2006 (2006-09-01), page S21, XP005741166 ISSN: 1090-3801**
- **LEDEBOER ANNEMARIE ET AL: "The glial modulatory drug AV(411) attenuates mechanical allodynia in rat models of neuropathic pain" NEURON GLIA BIOLOGY, vol. 2, no. Part 4, 2006, pages 279-291, XP009101145 ISSN: 1740-925X**
- **LEDEBOER ANNEMARIE ET AL: "Ibudilast (AV-411). A new class therapeutic candidate for neuropathic pain and opioid withdrawal syndromes." EXPERT OPINION ON INVESTIGATIONAL DRUGS JUL 2007, vol. 16, no. 7, July 2007 (2007-07), pages 935-950, XP002483491 ISSN: 1744-7658**
- **CAMPOS ADRIANA ROLIM ET AL: "Ketamine-induced potentiation of morphine analgesia in rat tail-flick test: Role of opioid-, alpha (2)-adrenoceptors and ATP-sensitive potassium channels", BIOLOGICAL & PHARMACEUTICAL BULLETIN, vol. 29, no. 1, January 2006 (2006-01), pages 86-89, ISSN: 0918-6158**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 131 841 B1

**Description**

**FIELD OF THE INVENTION**

The present invention relates generally to potentiation ofopioid-induced analgesia in a subject by administration of a phosphodiesterase (PDE) inhibitor. In particular, the present invention pertains to a PDE inhibitor, such as ibudilast (3-isobutyryl-2-isopropylpyrazolo[1,5-a]pyridine; also termed AV411 herein), in combination with an opioid analgesic, for use in treating acute pain.

**BACKGROUND OF THE INVENTION**

In recent years, pain management has become an area of increasing focus in the medical profession, partly due to the growing population of elderly, issues surrounding quality of life, and the growing numbers of patients reportedly suffering from pain. Pain is both a sensory and emotional experience, and is generally associated with tissue damage or inflammation. Typically, pain is divided into two general categories - acute pain and chronic pain. Both differ in their etiology, pathophysiology, diagnosis, and most importantly, treatment.

Morphine is a primary analgesic used in the treatment of pain as specified by the World Health Organization (WHO). In 1806, Friedrich Wilhelm Adam Sertürner started work on isolating the major active constituent of opium (Sertürner F., Darstellung der reinen Mohnsäure (Opiumsäure) nebst einer Chemischen Untersuchung des Opiums mir vorzüglicher Hinsicht auf einen darin neu entdeckten Stoff und die dahin gehörigen Bemerkungen. Journal der Pharmacie fuer Aerzte und Apotheker 14: 47-93, 1806) and named it morphine (after Morpheus, Ovid’s god of dreams, the son of sleep) (Huxtable RJ, Schwarz SK The isolation of morphine-first principles in science and ethics. Mol Interv. 2001 Oct; 1(4):189-91).

However, the chemical structure of morphine was not elucidated until early last century (Gulland JM, Robinson R: J. Chem. Soc. [London], 23, 980,1923). Since this time, many structural modifications have been made to morphine, producing semisynthetic and fully synthetic compounds with varied, but similar pharmacological actions.

Morphine belongs to a class of compounds know as 4,5-epoxymorphinans. Once the structure of morphine had been established, attempts to synthesize opioids that were more potent, longer acting and which had fewer side effects began. This gave rise to numerous other compound classes such as morphinans, benzomorphinans, arylmorphinans, 4-phenylpiperidines, 4-anilinopiperidines and 3,3-diphenylpropylamines, all of which have differing pharmacokinetic and pharmacodynamic properties. Some examples of these non-4,5-epoxymorphinans are levorphanol (morphinan), pethidine (4-phenylpiperidine), fentanyl (4-anilinopiperidine) and methadone (3,3-diphenylpropylamine).

The relief of pain by all of these clinically employed opioids is relatively selective in that other sensations (*e.g.*, touch, vision, hearing, smelling) are typically not compromised. However, the analgesic benefit of morphine and related opioids can be accompanied by other undesirable side effects involving the central nervous system and gastrointestinal system including drowsiness, respiratory depression, constipation, nausea and vomiting. There are significant adverse (respiratory depression) and unpleasant dose limiting side effects. Moreover, upon repeated use, a characteristic feature of opioid drugs is the development of tolerance and physical dependence ( Gilman, A.G., Goodman, L.S., Rall, T.W., and Murad, F. The Pharmacological Basis of Therapeutics. New York: MacMillan Publishing Co., 1985). Compounding the issue even further is evidence that opioid analgesics are ineffective in certain pain conditions (including certain neuropathic syndromes). The combination of dose limiting side effects and the potential development of tolerance which requires increases in analgesic requirements results in many patients who are left without adequate pain relief by opioids.

Given the broad, effective and recommended (WHO List of Essential Medicines, 14th Edition, March 2005) utility of opioid analgesics coupled with their limiting side effects described above, the identification of compounds which might improve opioid efficacy while allowing opioid dose-reduction has been pursued for many years. Some potentiators (enhanced efficacy of two agents, typically in a greater-than-additive manner) of opioid analgesia in rodent models include ketamine (AR Campos et al., Biol Pharm Bull, 29:86, 2006), dopamine antagonists (JA Kiritsy-Roy et al., Pharmacol Biochem Behav 32:717, 1989), and certain non-steroidal anti-inflammatory agents (S. Zelcer et al., Brain Res 1040:151, 2005).

The small molecule, ibudilast, (3-isoburyryl-2-isopropylpyrazolo[1,5-a]pyridine), is a non-selective inhibitor of cyclic nucleotide phosphodiesterase (PDE) (Fujimoto, T., et al., J. ofNeuroimmunology, 95 (1999) 35-92). Ibudilast also acts as an LTD4 antagonist, an anti-inflammatory, a PAF antagonist, and a vasodilatarory agent (Thompson Current Drug Reports). Ibudilast is thought to exert a neuroprotective role in the central nervous system of mammals, presumably via suppression of the activation of glial cells (Mizuno et al. (2004) Neuropharmacology 46: 404-411). Ibudilast has been widely used in Japan for relieving symptoms associated with ischemic stroke or bronchial asthma. Marketed indications for ibudilast in Japan include its use as a vasodilator, for treating allergy, eye tissue regeneration, ocular disease, and treatment of allergic ophthalmic disease (Thompson Current Drug Reports). In recent clinical trials, its use in the treatment of multiple selerosis, an inflammatory disease of the central nervous system, has been explored (News.Medical.Net;

Pharmaceutical News, 2 Aug 2005). While the use of ibudilast for a number of varying indications has been reported to date, to the best of the applicants' knowledge, its use as a potentiator of opioid analgesia in treating acute pain has heretofore remained unexplored.

From WO 2006/063048 it is known to use ibudilast for the treatment of chronic pain.

Further, WO 2007/146087 hints at the possibility of using PDE inhibitors for the treatment of pain but does not provide data on the treatment of acute pain, nor does it provide specific data for PDE-4 inhibitors in particular.

From Watkins et al., Trends in Neurosciences, Vol. 28 (12), Dec. 01, 2005, pp 661 to 669, it was known to overcome morphine tolerance and to treat chronic pain with PDE inhibitors.

Johnson et al, Eur. J of Pain, Saunders, London, GB, vol. 10 (1 Sept 2006), page S21 as well as Ledeboer et al, Neuron Glia Biol. Vol.2, Part 4, 2006, p. 279-291 teach that ibudilast is a potent suppressor of glial activation but do not disclose the use of PDE IV inhibitors for the treatment of acute pain.

In light of the shortcomings in current approaches for treating pain, there exists a need for improved compositions and methods for treating pain, particularly acute and subchronic pain and the associated symptoms.

## SUMMARY OF THE INVENTION

In one aspect, the invention provides a phosphodiesterase 4 inhibitor, as claimed in any of the claims as enclosed. In certain embodiments, the PDE inhibitor is selected from the group consisting of Rolipram, Arofylline, Roflumilast, Pentoxyfylline, and Propenrofylline. In a preferred embodiment, the phosphodiesterase-4 inhibitor is ibudilast. In certain embodiments, the opioid is morphine or oxycodone. In certain embodiments, the PDE inhibitor is administered either prior to or concurrently with the opioid.

In another aspect, the invention provides for the above use for treating acute pain comprising administering to a subject in need thereof a therapeutically effective amount of ibudilast and an opioid. In certain embodiments, the ibudilast is administered to the subject either prior to or concurrently with the opioid. If administered concurrently, the ibudilast and morphine can be in the same or different compositions. In certain embodiments, the opioid is morphine or oxycodone.

Mammalian subjects suitable for treatment by the methods described herein include, but are not limited to, those suffering from back pain, post-operative pain, injury-related pain, or disease-related pain. In one embodiment, the subject is a human.

In certain embodiments, ibudilast is administered systemically, for example, via intravenous, subcutaneous, oral, intranasal, sublingual or other systemic routes. In other embodiments, ibudilast is administered centrally, for example, intrathecally. In certain embodiments, ibudilast is administered orally or intraperitoneally. In one embodiment, the opioid is administered subcutaneously.

A therapeutic dosage amount of ibudilast or an opioid analgesic may be achieved by intermittent administration, or administration once daily *(i.e.,* in a single dose), twice daily *(i.e.,* in two separate doses), three times daily, or may be administered as multiple doses over a time course of several days, weeks, or even months. Such administering is typically over a duration of time effective to result in a diminution, and ideally elimination or even reversal, of acute pain. Exemplary durations of treatment include at least about one week, from 1 week to 1 month, from two weeks to 2 months, up to about 6 months, up to about 12 months or even longer. In one particular embodiment, treatment lasts from about 1 week to about 50 weeks. In a preferred embodiment of the treatment method, the administering is over a duration of time effective to result in elimination of acute pain.

In a further embodiment, the ibudilast and opioid analgesic are administered in combination with one or more other agents effective for treating pain. Such agents include analgesics, non-steroidal anti-inflammatory drugs (NSAIDs) and antidepressants. In various embodiments, one or more agents are selected from the group consisting of buprenorphine, naloxone, methadone, levomethadyl acerate, L-alpha acetylmethadol (LAAM), hydroxyzine, diphenoxylate, atropine, chlordiazepoxide, carbamazepine, mianserin, benzodiazepine, phenoziazine, disulfiram, acamprosate, topiramate, ondansetron, sertraline, bupropion, amantadine, amiloride, isradipine, tiagabine, baclofen, propranolol, tricyclic antidepressants, desipramine, carbamazepine, valproate, lamotrigine, doxepin, fluoxetine, imipramine, moclobemide, nortripryline, paroxetine, sertraline, tryptophan, venlafaxine, trazodone, quetiapine, zolpidern, zopiclone, zaleplon, gabapentin, memantine, pregabalin, cannabinoids, tramadol, duloxetine, milnacipran, naltrexone, paracetamol, metoclopramide, loperamide, clonidine, lofexidine, and diazepam.

In another aspect, the invention provides a composition or combination effective for treating acute pain. The composition comprises a combination of: (i) ibudilast, (ii) an opioid analgesic (*e.g.*, morphine, oxycodone, or related opioids), and (iii) optionally one or more additional agents effective for treating acute pain, wherein each of the components is either contained in a single composition or dosage form (such as in an admixture), or is present as a discrete or separate entity (*e.g.*, in a kit). A composition of the invention may optionally include one or more pharmaceutically acceptable excipients.

In yet another aspect, the present specification describes a kit comprising ibudilast, for the treatment of acute

pain, and additional agents effective for treating acute pain, for simultaneous, sequential or separate use, as well as instructions for use of the agents.

These and other embodiments of the subject invention will readily occur to those of skill in the art in view of the disclosure herein.

## BRIEF DESCRIPTION OF THE FIGURES

Figures IA-ID show rat tailflick and paw withdrawal responses in high and low intensity Hargreaves tests after administration of ibudilast (AV411) or vehicle alone. Tailflick responses (% maximal possible effect) on high (Figure 1A) and low (Figure 1B) intensity Hargreaves following 7.5 mg/kg ibudilast (■) or vehicle (o; 35% PEG) are shown. Paw withdrawal responses in high (Figure 1C) and low (Figure 1D) intensity Hargreaves tests following 7.5 mg/kg ibudilast (■) or vehicle (o; 35% PEG) are also shown.

Figures 2A-2D show that admnistration of ibudilast prior to morphine produces potentiated analgesia. Tailflick responses (% maximal possible effect) in high (Figure 2A) and low (Figure 2B) intensity Hargreaves tests following administration of 7.5 mg/kg ibudilast (■) or vehicle (o; 35% PEG) and 4 mg/kg morphine are shown. Paw withdrawal responses in high (Figure 2C) and low (Figure 2D) intensity Hargreaves tests following 7.5 mg/kg ibudilast (■) or vehicle (o; 35% PEG) and 4 mg/kg morphine are also shown.

Figures 3A and 3B shows measurements of the areas under the curves in Figures 2A-2D. The areas under the analgesia curves for tailflick (A) and paw (B) withdrawal responses following low and high intensity Hargreaves tests in animals that received 7.5 mg/kg ibudilast (black) or vehicle (white; 35% PEG) prior to administration of morphine are shown.

Figures 4A-4D show that administration of ibudilast prior to oxycodone produces potentiated analgesia. Tailflick responses (% maximal possible effect) in high (Figure 4A) and low (Figure 4B) intensity Hargreaves tests following 7.5 mg/kg ibudilast (■) or vehicle (o; 35% PEG) and oxycodone are shown. Paw withdrawal responses on high (Figure 4C) and low (Figure 4D) intensity Hargreaves following 7.5 mg/kg ibudilast (■) or vehicle (o; 35% PEG) and oxycodone are also shown.

Figures 5A and 5B shows measurements of the areas under the curves in Figures 4A-4D. Area under the analgesia curve for tailflick (Figure 5A) and paw (Figure 5B) withdrawal responses following low and high intensity Hargreaves in animals that received 7.5 mg/kg ibudilast (black) or vehicle (white; 35% PEG) are shown.

Figure 6 shows that concurrent oral administration of ibudilast and subcutaneous morphine potentiates morphine-induced analgesia. Rat tail-flick latency is shown as the percent maximum possible effect of a 15 second maximum tail flick test.

## DETAILED DESCRIPTION OF THE INVENTION

The practice of the present invention will employ, unless otherwise indicated, conventional methods of chemistry, biochemistry, and pharmacology, within the skill of the art. Such techniques are explained fully in the literature. See, e.g.; A.L. Lehninger, Biochemistry (Worth Publishers, Inc., current addition); Morrison and Boyd, Organic Chemistry (Allyn and Bacon, Inc., current addition); J. March, Advanced Organic Chemistry (McGraw Hill, current addition); Remington: The Science and Practice of Pharmacy, A. Gennaro, Ed., 20th Ed.; Goodman & Gilman The Pharmacological Basis of Therapeutics, J. Griffith Hardman, L. L. Limbird, A. Gilman, 10th Ed.

## I. DEFINITIONS

In describing and claiming the present invention, the following terminology will be used in accordance with the definitions described below.

It must be noted that, as used in this specification and the intended claims, the singular forms "a," "an," and "the" include plural referents unless the context clearly dictates otherwise. Thus, for example, reference to "a drug " includes a single drug as well as two or more of the same or different drugs, reference to "an optional excipient" refers to a single optional excipient as well as two or more of the same or different optional excipients, and the like.

"Pharmaceutically acceptable excipient or carrier" refers to an excipient that may optionally be included in the compositions of the invention and that causes no significant adverse toxicological effects to the patient.

"Pharmaceutically acceptable salt" includes, but is not limited to, amino acid salts, salts prepared with inorganic acids, such as chloride, sulfate, phosphate, diphosphate, hydrobromide, and nitrate salts, or salts prepared with an organic acid, such as malate, maleate, fumarate, tartrate, succinate, ethylsuccinate, citrate, acetate, lactate, methanesulfonate, benzoate, ascorbate, para-toluenesulfonate, palmoate, salicylate and stearate, as well as estolate, gluceptate

and lactobionate salts. Similarly salts containing pharmaceutically acceptable cations include, but are not limited to, sodium, potassium, calcium, aluminum, lithium, and ammonium (including substituted ammonium).

"Active molecule" or "active agent" as described herein includes any agent, drug, compound, composition of matter or mixture which provides some pharmacologic, often beneficial, effect that can be demonstrated *in-vivo* or *in vitro*. This includes foods, food supplements, nutrients, nutriceuticals, drugs, vaccines, antibodies, vitamins, and other beneficial agents. As used herein, the terms further include any physiologically or pharmacologically active substance that produces a localized or systemic effect in a patient.

"Substantially" or "essentially" means nearly totally or completely, for instance, 95% or greater of some given quantity.

"Optional" or "optionally" means that the subsequently described circumstance may or may not occur, so that the description includes instances where the circumstance occurs and instances where it does not.

By "acute pain" or "subchronic pain" is meant pain resulting from injury to the body and activation of nociceptive transducers at the site of local tissue damage in a subject. Such pain may result from any physical trauma, injury, surgery, or disease (*e.g.*, post-operative pain, back pain, bums, cancer or other pathological lesions). Acute pain refers to pain that occurs immediately following injury. Subchronic pain refers to prolonged pain following injury that may last a few days, weeks, or months until healing occurs.

"Nociception" is defined herein as pain sense. "Nociceptor" herein refers to a structure that mediates nociception. The nociception may be the result of a physical stimulus, such as, mechanical, electrical, thermal, or a chemical stimulus. Nociceptors are present in virtually all tissues of the body.

"Analgesia" is defined herein as the relief of pain without the loss of consciousness. An "analgesic" is an agent or drug useful for relieving pain, again, without the loss of consciousness.

The term "central nervous system" or "CNS" includes all cells and tissue of the brain and spinal cord of a vertebrate. Thus, the term includes, but is not limited to, neuronal cells, glial cells (astrocytes, microglia, oligodendrocytes), cerebrospinal fluid (CSF), interstitial spaces and the like.

"Glial cells" refer to various cells of the CNS also known as microglia, astrocytes, and oligodendrocytes.

The terms "subject", "individual" or "patient" are used interchangeably herein and refer to a vertebrate, preferably a mammal. Mammals include, but are not limited to, murines, rodents, simians, humans, farm animals, sport animals and pets.

The term "about", particularly in reference to a given quantity, is meant to encompass deviations of plus or minus five percent.

The terms "effective amount" or "pharmaceutically effective amount" of a composition or agent, as provided herein, refer to a nontoxic but sufficient amount of the composition to provide the desired response, such as suppression of glial activation in a subject, and optionally, a corresponding therapeutic effect, such as preventing, diminishing, or eliminating pain in a subject. The exact amount required will vary from subject to subject, depending on the species, age, and general condition of the subject, the severity of the condition being treated, the particular drug or drugs employed, mode of administration, and the like. An appropriate "effective" amount in any individual case may be determined by one of ordinary skill in the art using routine experimentation.

"Treatment" or "treating" acute or subchronic pain includes: (1) preventing pain, *i.e.* causing pain not To develop or to occur with less intensity in a subject that may be exposed to or predisposed to pain but does not yet experience or display pain, (2) inhibiting pain, *i.e.*, arresting the development or reversing pain, or (3) relieving pain, *i.e.*, decreasing the amount of pain experienced by the subject.

By "therapeutically effective dose or amount" of ibudilast is intended an amount that, when ibudilast is administered as described herein, brings about a positive therapeutic response in treatment of acute or subchronic pain, such as preventing, diminishing, or eliminating pain in a subject.

## II. MODES OF CARRYING OUT THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

The present invention is based on the discovery of a novel therapeutic methodology for safely and effectively treating acute pain using a PDE4 inhibitor, such as ibudilast, in combination with an opioid analgesic. As shown in Examples 1 and 2, ibudilast potentiates opioid-induced analgesic activity, thus, enhancing opioid-mediated pain relief in a subject. The use of a PDE inhibitor in combination with an opioid analgesic may permit a reduction in the opioid dosage needed to alieviate pain in a subject relative to the amount needed to produce an analgesic effect without administration of the PDE inhibitor, and accordingly, prevent or diminish adverse opioid side-effects, such as opioid

dependence and the symptoms of opioid withdrawal that often accompany treatment with opioids.

In order to further an understanding of the invention, a more detailed discussion is provided below regarding methods of treating acute pain using a PDE4 inhibitor in combination with an opioid analgesic.

## TREATMENT OF ACUTE AND SUBCHRONIC PAIN

The invention relates to the use of a PDE4- inhibitor, such as ibudilast, in combination with an opioid analgesic to treat acute pain in a subject. Ibudilast has been shown in the present application to potentiate opioid analgesic efficacy. In classic rat models of acute pain (paw and tail withdrawal responses to heat stimuli, administration of ibudilast in combination with an opioid analgesic (*e.g.*, morphine or oxycodone) unexpertedly yielded greater-than-additive (*i.e.*, potentiated or synergistic) analgesic efficacy (see Examples 1 and 2). Thus, ibudilast is identified, for the first time, as a potentiator of opioid-induced acute analgesic activity.

Exemplary PDE inhibitors other than ibudilast that may also be used to potentiate opioid-induced analgesic activity include, but are not limited to, Rolipram, Arofylline, Roflumilast, Pentoxyfylline, or Propentofylline.

Acute pain results from injury to the body and activation of nociceptive transducers at the site of local tissue damage in a subject. Such pain may result from *e.g.*, physical trauma, surgery, back spasms, dental procedures, such as molar extraction, bums, cancer, or other pathological lesions associated with disease. The methods of the invention are applicable to acute pain occurring immediately following an injury.

In certain embodiments, a therapeutically effective amount of a PDE4 inhibitor and a therapeutically effective amount of an opioid analgesic are administered to a subject in combination therapy with one or more other agents for treating pain. Such agents include, but are not limited to, analgesics, NSAIDs, benzodiazepines, and antidepressants. Exemplary agents include, but are not limited to, buprenorphine, naloxone, methadone, levomethadyl acetate, L-alpha acetylmethadol (LAAM), hydroxyzine, diphenoxylate, atropine, chlordiazepoxide, carbamazepine, mianserin, benzodiazepine, phenoziazine, disulfiram, acamprosate, topiramate, ondansetron, sertraline, bupropion, amantadine, amiloride, isradipine, tiagabine, baclofen, propranolol, tricyclic antidepressants, desipramine, carbamazepine, valproate, lamotrigine, doxepin, fluoxetine, imipramine, moclobemide, nortriptyline, paroxetine, sertraline, tryptophan, venlafaxine, trazodone, quetiapine, zolpidem, zopiclone, zaleplon, gabapentin, memantine, pregabalin, cannabinoids, tramadol, duloxetine, milnacipran, naltrexone, paracetamol, metoclopramide, loperamide, clonidine, lofexidine, and diazepam.

## PHARMACEUTICAL COMPOSITIONS FOR TREATING ACUTE OR SUBCHRONIC PAIN

### IBUDILAST

Ibudilast is a small molecule drug (molecular weight of 230.3) having the structure shown below.

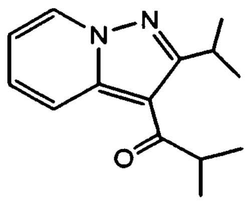

I

Ibudilast is also found under ChemBank ID 3227, CAS # 50847-11-5, and Beilstein Handbook Reference No. 5-24-03-00396. Its molecular formula corresponds to [$C_{14}H_{18}N_2O$]. Ibudilast is also known by various chemical names which include 2-methyl-1-(2-(1-methylethyl)pyrazolo(1,5-a)pyridin-3-yl)1-propanone; 3-isobutyryl-2-isopropylpyrazolo (1,5-a)pyridine]; and 1-(2-isopropyl-pyrazolo[1,5-a]pyridin-3-yl)-2-methyl-propan-1-one. Other synonyms for ibudilast include Ibudilastum (Latin), BRN 0656579, KC-404, and the brand name Ketas®. Ibudilast, as referred to herein, is meant to include any and all pharmaceutically acceptable salt forms thereof, prodrug forms (*e.g.*, the corresponding ketal), and the like, as appropriate for use in its intended formulation for administration.

Ibudilast is a non-selective nucleotide phosphodiesterase (PDE) inhibitor (most active against PDE 3, PDE4, PDE 10, and PDE 11 (Gibson ct al. (2006) Eur. J. Pharmacology 538:39-42)), and has also been reported to have LTD4 and PAF antagonistic activities. Its profile appears effectively anti-inflammatory and unique in comparison to other PDE inhibitors and anti-inflammatory agents. PDEs catalyze the hydrolysis of the phosphoester bond on the 3'-carbon to yield the corresponding 5'-nucleotide monophosphare. Thus, they regulate the cellular concentrations of cyclic nucleotides. Since extracellular receptors for many hormones and neurotransmitters utilize cyclic nucleotides as second messengers, the PDEs also regulate cellular responses to these extracellular signals. There are 11 families of PDEs: $Ca^{2+}$/calmodulin-

dependent PDEs (PDE1); cGMP-stimulated PDEs (PDE2); cGMP-inhibited PDEs (PDE3); cAMP-specific PDEs (PDE4); cGMP-binding PDEs (PDEs5); photoreceptor PDEs (PDE6); high affinity, cAMP-specific PDEs (PDE7); specific PDE (PDE8); high affinity cGMP-specific PDEs (PDE9); and mixed cAMP and cGMP PDEs (PDE 10, PDE11).

As stated previously, a reference to any one or more of the herein-described drugs, in particular ibudiiast, is meant to encompass, where applicable, any and all enanriomers, mixtures of enantiomers including racemic mixtures, prodrugs, pharmaceutically acceptable salt forms, hydrates (*e.g.*, monohydrates, dihydrates, etc.), different physical forms (*e.g.*, crystalline solids, amorphous solids), metabolites, and the like.

## FORMULATION COMPONENTS

In addition to comprising a PDE4' inhibitor, such as ibudilast, the compositions of the invention may optionally contain one or more additional components as described below.

### Excipients/Carriers

In addition to a PDE4 inhibitor, the compositions of the invention for treating acute pain may further comprise one or more pharmaceutically acceptable excipients or carriers. Exemplary excipients include, without limitation, polyethylene glycol (PEG), hydrogenated castor oil (HCO), cremophors, carbohydrates, starches (*e.g.*, corn starch), inorganic salts, antimicrobial agents, antioxidants, binders/fillers, surfactants, lubricants (*e.g.*, calcium or magnesium stearate), glidants such as talc, disintegrants, diluents, buffers, acids, bases, film coats, combinations thereof, and the like.

A composition of the invention may include one or more carbohydrates such as a sugar, a derivatized sugar such as an alditol, aldonic acid, an esterified sugar, and/or a sugar polymer. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like.

Also suitable for use in the compositions of the invention are potato and corn-based starches such as sodium starch glycolate and directly compressible modified starch.

Further representative excipients include inorganic salt or buffers such as citric acid, sodium chloride, potassium chloride, sodium sulfate, potassium nitrate, sodium phosphate monobasic, sodium phosphate dibasic, and combinations thereof.

A composition comprising a PDE4 inhibitor may also include an antimicrobial agent, *e.g.*, for preventing or deterring microbial growth. Non-limiting examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

A composition comprising a PDE4 inhibitor may also contain one or more antioxidants. Antioxidants are used to prevent oxidation, thereby preventing the deterioration of the drug(s) or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

Additional excipients include surfactants such as polysorbates, *e.g.*, TWEEN 20 and TWEEN 80, and pluronics such as F68 and F88 (both of which are available from

BASF, Mount Olive, New Jersey), sorbitan esters, lipids (*e.g.*, phospholipids such as lecithin and other phosphatidylcholines, and phosphatidyluthanolamines), fatty acids and fatty esters, steroids such as cholesterol, and chelating agents, such as EDTA, zinc and other such suitable cations.

Further, a composition comprising a PDE4 inhibitor may optionally include one or more acids or bases. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof.

The amount of any individual excipient in the composition will vary depending on the role of the excipient, the dosage requirements of the active agent components, and particular needs of the composition. Typically, the optimal amount of any individual excipient is determined through routine experimentation, *i.e.*, by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99%

by weight, preferably from about 5% to about 98% by weight, more preferably from about 15 to about 95% by weight of the excipient. In general, the amount of excipient present in a 3,4,6-substituted pyridazine composition is selected from the following: at least about 2%, 5%, 10%, 15%, 20%, 25%, 30%, 35%, 40%, 45%, 50%, 55%, 60%, 65%, 70%, 75%, 80%, 85%, 90%, or even 95% by weight.

These foregoing pharmaceutical excipients along with other excipients are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D-C., 2000.

## Other Actives

A described above, the formulation (or kit) in accordance with the invention may contain, in addition to a PDE4 inhibitor, one or more opioid analgesics, and optionally additional active agents effective in treating acute or subchronic pain, including, but not limited to, other analgesics, NSAIDs, benzodiazepines and antidepressants. Such actives include morphine, oxycodone, and related opiates, buprenorphine, naloxone, methadone, levomethadyl acetate, L-alpha acetylmethadol (LAAM), hydroxyzine, diphenoxylate, atropine, chlordiazepoxide, carbamazepine, mianserin, benzodiazepine, phenoziazine, disulfiram, acamprosate, topiramate, ondansetron, sertraline, bupropion, amantadine, amiloride, isradipine, tiagabine, baclofen, propranolol, tricyclic antidepressants, desipramine, carbamazepine, valproate, lamotrigine, doxepin, fluoxetine, imipramine, moclobemide, nortriptyline, paroxetine, sertraline, tryptophan, venlafaxine, trazodone, quetiapine, zolpidem, zopiclone, zaleplon, gabapentin, memantine, pregabalin, cannabinoids, tramadol, duloxetine, milnacipran, naltrexone, paracetamol, meroclopramide, loperainide, clonidine, lofexidine, and diazepam.

The opiates, morphine and oxycodone, elicit their effects by activating opiate receptors that are widely distributed throughout the brain and body. Once an opiate reaches the brain, it quickly activates the opiate receptors found in many brain regions and produces an effect that correlates with the area of the brain involved. There are several types of opiate receptors, including the delta, mu, and kappa receptors. Opiates and endorphins function to block pain signals by binding to the mu receptor site.

Gabapentin, also known as Neurontin®, is Structurally related to the neurotransmitter GABA. Although structurally related to GABA, gabapenrin does not interact with GABA receptors, is nor converted metabolically into GABA or a GABA agonist, and is not an inhibitor of GABA uptake or degradation. Gabapentin has no activity at GABAA or GABAB receptors of GABA uptake carriers of the brain, but instead interacts with a high-affinity binding site in brain membranes (an auxiliary subunit of voltage-sensitive $Ca^{2+}$ channels). The exact mechanism of action is unknown, only that its physiological site of action is the brain. The structure of gabapentin allows it to pass freely through the blood-brain barrier. *In vitro,* gabapentin has many pharmacological actions including modulating the action of the GABA synthetic enzyme, increasing non-synaptic GABA responses from neural tissue, and reduction of the release of several mono-amine neurotransmitters. Daily dosages of gabapentin typically range from about 600 to 2400 mg/day, more preferably from about 900 to 1800 mg/day, and are administered in divided doses, for example, three times a day. Conventional unit dosage forms are 300 or 400 mg capsules or 600 or 800 mg tablets.

The active agent, memantine, is a receptor antagonist. Memantine is believed to function as a low to moderate affinity uncompetitive (open-channel) NMDA receptor antagonist which binds to the NMDA receptor-operated cation channels. Recommended daily dosage amounts typically range from about 5 mg to 20 mg.

The cannabinoids, *e.g.*, tetrahydrocannabinol, bind to the cannabinoid receptor referred to as $CB_1$. $CB_1$ receptors are found in brain and peripheral tissues; $CB_1$ receptors are present in high quantities in the central nervous system, exceeding the levels of almost all neurotransmitter receptors. An additional cannabinoid receptor subtype termed 'CB2' has also been identified. See, *e.g.*, Martin, B.R., et al., The Journal of Supportive Oncology, Vol. 2, Number 4, July/August 2004.

Although its mechanism of action has not yet been fully elucidated, the opioid, tramadol, is believed to work through modulation of the GABAergic, noradrenergic and serotonergic systems. Tramadol, and its metabolite, known as M1, have been found to bind to $\mu$-opioid receptors (thus exerting its effect on GABAergic transmission), and to inhibit re-uptake of 5-HT and noradrenaline. The second mechanism is believed to contribute since the analgesic effects of tramadol are not fully antagonised by the $\mu$-opioid receptor antagonist naloxone. Typical daily dosages range from about 50 to 100 milligrams every 4 to 6 hours, with a total daily dosage not to exceed 400 milligrams.

Lamotrigine is a phenyltriazine that stabilizes neuronal membranes by blocking voltage-sensitive sodium channels, which inhibit glutamate and aspartate (excitatory amino acid neurotransmitter) release. The daily dosage of lamotrigine typically ranges from 25 milligrams per day to 500 mg per day. Typical daily dosage amounts include 50 mg per day, 100 mg per day, 150 mg per day, 200 mg per day, 300 mg per day, and 500 mgs per day, not exceed 700 mgs per day.

Carbamazepine acts by blocking voltage-sensitive sodium channels. Typical adult dosage amounts range from 100-200 milligrams one or two times daily, to an increased dosage of 800-1200 milligrams daily generally administered in 2-3 divided doses.

Duloxetine is a potent inhibitor of neuronal uptake of serotonin and norephinephrine and a weak inhibitor of dopamine re-uptake. Typical daily dosage amounts range from about 40 to 60 milligrams once daily, or 20 to 30 milligrams twice daily.

Milnacipran acts as a serotonin and norepinephrine reuptake inhibitor. Daily dosage amounts typically range from about 50 to 100 milligrams once or twice daily.

The dosage amounts provided above are meant to be merely guidelines; the precise amount of a secondary active agent to be administered during combination therapy with ibudilast will, of course, be adjusted accordingly and will depend upon factors such as inrendcd patient population, the particular acute or subchronic pain symptom or condition to be treated, potential synergies between the active agents administered, and the like, and will readily be determined by one skilled in the art based upon the guidance provided herein.

**Sustained Delivery Formulations**

The compositions may also be formulated in order to improve stability and extend the half-life of the PDE4 inhibitor. For example, the PDE inhibitor may be delivered in a sustained-release formulation. Controlled or sustained-release formulations are prepared by incorporating ibudilast into a carrier or vehicle such as liposomes, nonresorbable impermeable polymers such as ethylenevinyl acetate copolymers and Hytrel® copolymers. swellable polymers such as hydrogels, or resorbable polymers such as collagen and certain polyacids or polyesters such as those used to make resorbable sutures. Additionally, the PDE, inhibitor can be encapsulated, adsorbed to, or associated with, particulate carriers. Examples of particulate carriers include those derived from polymethyl methacrylate polymers, as well as microparticles derived from poly(lactides) and poly(lactide-co-glycolides), known as PLG. See, *e.g.,* Jeffery et al., Pharm. Res. (1993) 10:62-368; and McGee et al., J. Microencap. (1996).

**DELIVERY FORMS**

The compositions comprising a PDE inhibitor described herein encompass all types of formulations, and in particular, those that are suited for systemic or intrathecal administration. Oral dosage forms include tablets, lozenges, capsules, syrups, oral suspensions, emulsions, granules, and pellets. Alternative formulations include aerosols, transdermal patches, gels, creams, ointments, suppositories, powders or lyophilates that can be reconstituted, as well as liquids. Examples of suitable diluents for reconstituting solid compositions, *e.g.*, prior to injection, include bacteriostatic water for injection, dextrose 5% in water, phosphate-buffered saline, Ringer's solution, saline, sterile water, deionized water, and combinations thereof. With respect to liquid pharmaceutical compositions, solutions and suspensions are envisioned. Preferably, a composition comprising a PDE4 inhibitor is one suited for oral administration.

In turning now to oral delivery formulations, tablets can be made by compression or molding, optionally with one or more accessory ingredients or additives. Compressed tablets are prepared, for example, by compressing in a suitable tabletting machine, the active ingredients in a free-flowing form such as a powder or granules, optionally mixed with a binder (*e.g.*, povidone, gelatin, hydroxypropylmethyl cellulose), lubricant, inert diluent, preservative, disintegrant (*e.g.*, sodium starch glycolate, cross-linked povidone, cross-linked sodium carboxymethyl cellulose) and/or surface-active or dispersing agent.

Molded tablets are made, for example, by molding in a suitable tabletting machine, a mixture of powdered compounds moistened with an inert liquid diluent. The tablets may optionally be coated or scored, and may be formulated so as to provide slow or controlled release of the active ingredients, using, for example, hydroxypropylmethyl cellulose in varying proportions to provide the desired release profile. Tablets may optionally be provided with a coating, such as a thin film, sugar coating, or an enteric coating to provide release in parts of the gut other than the stomach. Processes, equipment, and toll manufacturers for tablet and capsule making are well-known in the art.

Formulations for topical administration in the mouth include lozenges comprising the active ingredients, generally in a flavored base such as sucrose and acacia or tragacanth and pastilles comprising the active ingredients in an inert base such as gelatin and glycerin or sucrose and acacia.

A pharmaceutical composition for topical administration may also be formulated as an ointment, cream, suspension, lotion, powder, solution, paste, gel, spray, aerosol or oil.

Alternatively, the formulation may be in the form of a patch (*e.g.*, a transdermal patch) or a dressing such as a bandage or adhesive plaster impregnated with active ingredients and optionally one or more excipients or diluents. Topical formulations may additionally include a compound that enhances absorption or penetration of the ingredients through the skin or other affected areas, such as dimethylsulfoxidem bisabolol, oleic acid, isopropyl myristate, and D-limonene, to name a few.

For emulsions, the oily phase is constituted from known ingredients in a known manner. While this phase may comprise merely an emulsifier (otherwise known as an emulgent), it desirably comprises a mixture of at least one emulsifier with a fat and/or an oil. Preferably, a hydrophilic emulsifier is included together with a lipophilic emulsifier that

acts as a stabilizer. Together, the emulsifier(s) with or without stabilizer(s) make up the so-called emulsifying wax, and the wax together with the oil and/or fat make up the so-called emulsifying ointment base which forms the oily dispersed phase of cream formulations. Illustrative emulgents and emulsion stabilizers include TWEEN 60, SPAN 80, cetostearyl alcohol, myristyl alcohol, glyceryl monostearate and sodium lauryl sulfate.

Formulations for rectal administration are typically in the form of a suppository with a suitable base comprising, for example, cocoa butter or a salicylate.

Formulations suitable for vaginal administration generally take the form of a suppository, tampon, cream, gel, paste, foam or spray.

Formulations suitable for nasal administration, wherein the carrier is a solid, include a coarse powder having a particle size, for example, in the range of about 20 to about 500 microns. Such a formulation is typically administered by rapid inhalation through the nasal passage, *e.g.*, from a container of the powder held in proximity to the nose. Alternatively, a formulation for nasal delivery may be in the form of a liquid, *e.g.*, a nasal spray or nasal drops.

Aerosolizable formulations for inhalation may be in dry powder form (*e.g.*, suitable for administration by a dry powder inhaler), or, alternatively, may be in liquid form, *e.g.*, for use in a nebulizer. Nebulizers for delivering an aerosolized solution include the AERx™ (Aradigm), the Ultravent® (Mallinkrodt), and the Acorn II® (Marquest Medical Products). A composition of the invention may also be delivered using a pressurized, metered dose inhaler (MDI), *e.g.*, the Ventolin® metered dose inhaler, containing a solution or suspension of a combination of drugs as described herein in a pharmaceutically inert liquid propellant, *e.g.*, a chlorofluorocarbon or fluorocarbon.

Formulations suitable for parenteral administration include aqueous and nonaqueous isotonic sterile solutions suitable for injection, as well as aqueous and nonaqueous sterile suspensions.

Parenteral formulations are optionally contained in unit-dose or multi-dose sealed containers, for example, ampoules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the types previously described.

A formulation for use with the invention may also be a sustained release formulation, such that each of the drug components is released or absorbed slowly over time, when compared to a non-sustained release formulation. Sustained release formulations may employ pro-drug forms of the active agent, delayed-release drug delivery systems such as liposomes or polymer matrices, hydrogels, or covalent attachment of a polymer such as polyethylene glycol to the active agent.

In addition to the ingredients particularly mentioned above, the formulations may optionally include other agents conventional in the pharmaceutical arts and particular type of formulation being employed, for example, for oral administration forms, the composition for oral administration may also include additional agents as sweeteners, thickeners or flavoring agents.

The compositions comprising a PDE4 inhibitor may also be prepared in a form suitable for veterinary applications.

## KITS

Also provided herein is a kit containing a composition comprising a PDE4 inhibitor (*e.g.*, ibudilast), accompanied by instructions for use, *e.g.*, in treating acute pain. In addition, the kit may optionally contain an opioid analgesic (*e.g.*, morphine or oxycodone) or one or more other agents for treating pain.

For example, in instances in which each of the drugs themselves are administered as individual or separate dosage forms, the kit comprises a PDE4 inhibitor in addition to each of the drugs making up the composition of the invention, along with instructions for use. A PDE inhibitor and one or more opioids or other agents may be present in the same or separate compositions. The drug components may be packaged in any manner suitable for administration, so long as the packaging, when considered along with the instructions for administration, clearly indicates the manner in which each of the drug components is to be administered.

For example, for an illustrative kit comprising ibudilast and morphine, the kit may be organized by any appropriate time period, such as by day. As an example, for Day I, a representative kit may comprise unit dosages of each of ibudilast and morphine. If each of the drugs is to be administered twice daily, then the kit may contain, corresponding to Day 1, two rows of unit dosage forms of each of ibudilast and morphine, along with instructions for the timing of administration. Alternatively, if one or more of the drugs differs in the timing or quantity of unit dosage form to be administered in comparison to the other drug members of the combination, then such would be reflected in the packaging and instructions. Various embodiments according to the above may be readily envisioned, and would of course depend upon the particular combination of drugs, in addition to the PDE4 inhibitor, employed for treatment, their corresponding dosage forms, recommended dosages, intended patient population. The packaging may be in any form commonly employed for the packaging of pharmaceuticals, and may utilize any of a number of features such as different colors, wrapping, ramper-resistant packaging, blister paks, dessicants, and the like.

## METHOD OF ADMINISTRATION

As set forth above, the present invention encompasses a method of treating a mammalian subject suffering from acute pain by administering therapeutically effective dosages of a PDE4 inhibitor and an opioid analgesic. Such administering is effective to decrease the amount of pain experienced by the subject, *i.e.*, to result in significant attenuation or elimination of acute pain.

Therapeutic amounts can be empirically determined and will vary with the particular condition being treated, the subject, the specific opioid analgesic, and the particular efficacy and toxicity of each of the active agents contained in the composition. The actual dose to be administered will vary depending upon the age, weight, and general condition of the subject as well as the severity of the condition being treated, the judgment of the health care professional, and particular mode of administration.

The method of the invention may, in certain instances, comprise a step of selecting a subject experiencing acute or subchronic pain prior to administering thereto ibudilast and an analgesic opioid. Such subjects are typically selected from those suffering from physical trauma associated with injury, surgery, or disease, (e.g., post-operative pain, back spasms, bums, cancer, or pathological lesions).

The method of the invemion may be effective to not only significantly attenuate acute pain, but to even reverse it, such that the resulting pain relief is long-lasting. Thus, the administering of a PDE4 inhibitor in combination with an opioid analgesic as described herein may be effective to result in sustained attenuation of acute pain for an overnight duration. For example, therapeutically effective doses of a PDE inhibitor and an opioid analgesic may be effective to treat acute pain for a duration of up to at least 4 hours, 6 hours, 8 hours, 10 hours, 12 hours, 16 hours, 18 hours or even 20 hours or greater.

The PDE inhibitor and an opioid analgesic may also be administered in combination with one or more additional agents effective for treating acute pain. Exemplary agents include other analgesics, non-steroidal anti-inflammatory drugs (NSAIDs), benzodiazepines and antidepressants.

Preferred methods of delivery of therapeutic formulations comprising a PDE4 inhibitor for the treatment of acute pain include systemic and localized delivery, *i.e.*, directly into the central nervous system. Such routes of administration include but are not limited to, oral, intra-arterial, intrathecal, intraspinal, intramuscular, subcutaneous, intraperitoneal, intravenous, intranasal, and inhalation routes.

More particularly, a formulation containing a PDE4 inhibitor of the present invention may be administered for therapy by any suitable route, including without limitation, oral, rectal, nasal, topical (including transdermal, aerosol, buccal and sublingual), vaginal, parenteral (including subcutaneous, intramuscular, intravenous and intradermal), intrathecal, and pulmonary. The preferred route will, of course, vary with the condition and age of the recipient, the particular neuralgia-associated syndrome being treated, and the specific combination of drugs employed.

One preferred mode of administration for delivery of a PDE4 inhibitor is directly to neural tissue such as peripheral nerves, the retina, dorsal root ganglia, neuromuscular junction, as well as the CNS, *e.g.*, to target spinal cord glial cells by injection into, *e.g.*, the ventricular region, as well as to the striatum (*e.g.*, the caudate nucleus or putamen of the striatum), spinal cord and neuromuscular junction, with a needle, catheter or related device, using neurosurgical techniques known in the art, such as by stereotactic injection (see, *e.g.,* Stein et al., J. Virol. 73:3424-3429, 1999; Davidson et al., PNAS 97:3428-3432, 2000 ; Davidson et al., Nat.Genet. 3:219-223, 1993; and Alisky and Davidson, Hum. Gene Ther. 11:2315-2329, 2000).

A particularly preferred method for targeting spinal cord glia is by intrathecal delivery, rather than into the cord tissue itself.

Another preferred method for administering the compositions comprising a PDE4 inhibitor of the invention is by delivery to dorsal root ganglia (DRG) neurons, *e.g.*, by injection into the epidural space with subsequent diffusion to DRG. For example, an ibudilast-based composition can be delivered via intrathecal cannulation under conditions where ibudilast is diffused to DRG. See, *e.g.,* Chiang et al., Acta Anaesthesiol. Sin. (2000) 38:31-36; Jain, K.K., Expert Opin. Investig. Drugs (2000) 9:2403-2410.

Yet another mode of administration to the CNS uses a convection-enhanced delivery (CED) system. In this way, the PDE inhibitor can be delivered to many cells over large areas of the CNS. Any convection-enhanced delivery device may be appropriate for delivery of ibudilast. In a preferred embodiment, the device is an osmotic pump or an infusion pump. Both osmotic and infusion pumps are commercially available from a variety of suppliers, for example Alzet Corporation, Hamilton Corporation, Alza, Inc., Palo Also, California). Typically, a composition comprising a PDE inhibitor or glial attenuator of the invention is delivered via CED devices as follows. A catheter, cannula or other injection device is inserted into CNS tissue in the chosen subject. Stereotactic maps and positioning devices are available, for example from ASI Instruments, Warren, MI. Positioning may also be conducted by using anatomical maps obtained by CT and/or MRI imaging to help guide the injection device to the chosen target. For a detailed description regarding CED delivery, see U.S. Patent No. 6,309,634.

A composition comprising a PDE4 inhibitor, when comprising more than one active agent, may be administered

as a single combination composition comprising a combination of the PDE4 inhibitor and at least one additional active agent effective in the treatment of acute or subchronic pain. In terms of patient compliance and ease of administration, such an approach is preferred, since patients are often adverse to taking multiple pills or dosage forms, often multiple times daily, over the duration of treatment. Alternatively, albeit less preferably, the combination of the invention is administered as separate dosage forms. In instances in which the drugs comprising the therapeutic composition of the invention arc administered as separate dosage forms and co-administration is required, the PDE4 inhibitor and each of the additional active agents may be administered simultaneously, sequentially in any order, or separately.

## DOSAGES

Therapeutic amounts can be empirically determined by those skilled in the art and will vary with the particular condition being treated, the subject, the particular opioid employed, and the efficacy and toxicity of each of the active agents contained in the composition. The actual dose to be administered will vary depending upon the age, weight, and general condition of the subject as well as the severity of the condition being treated, the judgment of the health care professional, and the particular combination of opioid, PDE inhibitor, and any other agents being administered.

Therapeutically effective amounts can be determined by those skilled in the art, and will be adjusted to the requirements of each particular case. Generally, a therapeutically effective amount of a PDE inhibitor will range from a total daily dosage, for example in humans, of about 0.1 and 500 mg/day, more preferably, in an amount between I and 200 mg/day, I and 100 mg/day, I and 40 mg/day, or 1 and 20 mg/day, administered as either a single dosage or as multiple dosages.

Depending upon the dosage amount and precise condition to be treated, administration can be one, two, or three times daily, or even more, for a time course of one day to several days, weeks, months, and even years, and may even be for the life of the patient. Intermittent dosing may also be employed, *e.g.*, in response to acute or subchronic pain, with a maximal dose not to be exceeded as recommended by the practicing physician. Illustrative dosing regimes will last a period of at least about a week, from about 1-4 weeks, from 1-3 months, from 1-6 months, from 1-50 weeks, from 1-12 months, or longer,

## PAIN MODELS

The ability of a PDE inhibitor in combination with an opioid analgesic to treat acute pain can be evaluated by any of the standard pain models known in the art. Examples of such models are as follows.

Tail Flick Model: The tail-flick test (D'Amour et al. (1941) J. Pharmacol. Exp. and Ther. 72:74-79) is a model of acute pain. A towel-wrapped rat is placed on a test stage such that a focused light source beams on the dorsal surface of the rat's tail. A photosensor is present on the test stage located opposite the light source and below the rat's tail. To begin the test, the rat's tail blocks the light, thus preventing the light reaching the photosensor. Latency measurement begins with the activation of the light source. When a rat moves or flicks its tail, the photosensor detects the light source and stops the measurement. The test measures the period of time (duration) that the rat's tail remains immobile (latent). Rats are tested prior to administration thereto of a compound of interest and then at various times after such administration. The light source is set to an intensity that produced a tail response latency of about 3 seconds when applied to the tails of rats to which no compound has been administered.

Rat Tail Immersion Model: The rat tail immersion assay is also a model of acute pain. A rat is loosely held in hand while covered with a small folded thin cotton towel with its tail exposed. The tip of the tail is dipped into a, e.g., 52°C water bath to a depth of two inches. The rat responds by either wiggling of the tail or withdrawal of the tail from the water; either response is scored as the behavioral end-point. Rats are tested for a tail response latency (TRL) score prior to administration thereto of a compound of interest and then retested for TRL at various times after such administration.

Carrageenan-induced Paw Hyperalgesia Model: The carrageenan paw hyperalgesia test is a model of inflammatory pain. A subcutaneous injection of carrageenan is made into the left hindpaws of rats. The rats are treated with a selected agent before, e.g., 30 minutes, the carrageenan injection or after, e.g., two hours after, the carrageenan injection. Paw pressure sensitivity for each animal is tested with an analgesymeter three hours after the carrageenan injection. See, Randall et al. (1957) Arch. Int. Pharmacodyn. 111:409-419.

The effects of selected agents on carrageenan-induced paw edema can also be examined. This test (see, Vinegar et al. (1969) J. Phamacol. Exp. Ther. 166:96-103) allows an assessment of the ability of a compound to reverse or prevent the formation of edema evoked by paw carrageenan injection. The paw edema test is carried out using a plethysmometer for paw measurements. After administration of a selected agent, a carrageenan solution is injected subcutaneously into the lateral foot pad on the plantar surface of the left hind paw. At three hours post-carrageenan treatment, the volume of the treated paw (left) and the un-treated paw (right) is measured using a plethysmometer.

Formalin Behavioral Response Model: The formalin test is a model of acute, persistent pain. Response to

formalin treatment is biphasic (Dubuisson et al. (1977) Pain 4:161-174). The Phase I response is indicative of a pure nociceptive response to the irritant. Phase 2, typically beginning 20 to 60 minutes following injection of formalin, is thought to reflect increased sensitization of the spinal cord.

Von Frey Filament Test: The effect of compounds on mechanical allodynia can be determined by the von Frey filament test in rats with a tight ligation of the L-5 spinal nerve: a model of painful peripheral neuropathy. The surgical procedure is performed as described by Kim et al. (1992) Pain 50:355-363. A calibrated series of von Frey filaments are used to assess mechanical allodynia (Chaplan et al. (1994) J. Neurosci. Methods 53:55-63). Filaments of increasing stiffness are applied perpendicular to the midplantar surface in the sciatic nerve distribution of the left hindpaw. The filaments are slowly depressed until bending occurred and are then held for 4-6 seconds. The filament application order and number of trials were determined by the up-down method of Dixon (Chaplan et al., *supra).* Flinching and licking of the paw and paw withdrawal on the ligated side are considered positive responses.

Chronic Constriction Injury: Heat and cold allodynia responses can be evaluated as described below in rats having a chronic constriction injury (CCI). A unilateral mononeuropathy is produced in rats using the chronic constriction injury model described in Bennett et al. (1988) Pain 33:87-107.

CCI is produced in anesthetized rats as follows. The lateral aspect of each rat's hind limb is shaved and scrubbed with Nolvasan. Using aseptic techniques, an incision is made on the lateral aspect of the hind limb at the mid-thigh level. The biceps femoris is bluntly dissected to expose the sciatic nerve. On the right hind limb of each rat, four loosely tied ligatures (for example, Chromic gut 4.0; Ethicon, Johnson and Johnson, Somerville, NJ) are made around the sciatic nerve approximately 1-2 mm apart. On the left side of each rat, an identical dissection is performed except that the sciatic nerve is not ligated (sham). The muscle is closed with a continuous suture pattern with, e.g., 4-0 Vicryl (Johnson and Johnson, Somerville, NJ) and the overlying skin is closed with wound clips. The rats are ear-tagged for identification purposes and returned to animal housing.

Radiant Heat Model: CCI rats are tested for thermal hyperalgesia at least 10 days post-op. The test apparatus consists of an elevated heated (80-82°F) glass platform. Eight rats at a time, representing all testing groups, are confined individually in inverted plastic cages on the glass floor of the platform at least 15 minutes before testing. A radiant heat source placed underneath the glass is aimed at the plantar hind paw of each rat. The application of heat is continued until the paw is withdrawn (withdrawal latency) or the time elapsed is 20 seconds. This trial is also applied to the sham operated leg. Two to four trials are conducted on each paw, alternately, with at least 5 minutes interval between trials. The average of these values represents the withdrawal latency.

Cold Allodynia Model: The test apparatus and methods of behavioral testing is described in Gogas et al. (1997) Analgesia 3:111-118. The apparatus for testing cold allodynia in neuropathic (CCI) rats consists of a Plexiglass chamber with a metal plate 6 cm from the bottom of the chamber. The chamber is filled with ice and water to a depth of 2.5 cm above the metal plate, with the temperature of the bath maintained at 0-4°C throughout the test.

Chung Model of Rat Neuropathic Pain: Heat and cold allodynia responses as well as mechanical allodynia sensations can be evaluated as described below in rats following spinal nerve injury (*e.g.* ligation, transaction). Details are as initially described in SH Kim and JM Chung, Pain (1992) 50:355-363.

The Hargreaves Test: The Hargreaves test (Hargreaves et al., Pain (1998) 32:77-88) is also a radiant heat model for pain. CCI rats are tested for thermal hyperalgesia at least 10 days post-op. The test apparatus consists of an elevated heated (80-82°F) glass platform. Eight rats at a time, representing all testing groups, are confined individually in inverted plastic cages on the glass floor of the platform at least 15 minutes before testing. A radiant heat source placed underneath the glass is aimed at the plantar hind paw of each rat. The application of heat is continued until the paw is withdrawn (withdrawal latency) or the time elapsed is 20 seconds. This trial is also applied to the sham operated leg. Two to four trials are conducted on each paw, alternately, with at least 5 minutes interval between trials. The average of these values represents the withdrawal latency.

## III. EXPERIMENTAL

Below are examples of specific embodiments for carrying out the present invention. The examples are offered for illustrative purposes only, and are not intended to limit the scope of the present invention in any way.

Efforts have been made to ensure accuracy with respect to numbers used (*e.g.*, amounts, temperatures, etc.), but some experimental error and deviation should, of course, be allowed for.

EXAMPLE 1

TREATMENT WITH IBUDILAST INTRAPERITONEALLY SHORTLY BEFORE SUBCUTANEOUS ADMINISTRATION OF MORPHINE OR OXYCODONE

**A. Materials and Methods**

**Animals**

Pathogen-free adult male Sprague-Dawley rats (300-375 g; Harlan Labs, Madison, WI) were used in all experiments. Rats were housed in temperature (23±3 °C) and light (12 h:12 h light:dark cycle; lights on at 0700) controlled rooms with standard rodent chow and water available ad libitum. All procedures were approved by the Institutional Animal Care and Use Committee of the University of Colorado at Boulder. Each study represents an n = 6 per group.

**Drugs**

Morphine sulfate and oxycodone hydrochloride were purchased from Sigma (St. Louis, MO, USA). Morphine and oxycodone were administered subcutaneously at 4 mg/kg in a dose volume of 1 ml/kg in sterile injection normal saline. Ibudilast was supplied by Avigen (Alameda, CA, USA). Ibudilast was administered intraperitoneally at 7.5 mg/kg in a dose volume of 2.5 ml/kg in 35% PEG: 65% injection saline. Morphine and oxycodone doses reported are as free base concentrations.

**Behavioral Measures**

*Hargreaves tests for analgesia and hyperalgesia.* Rats received at least three 60 min habituations to the test environment prior to behavioral testing. Thresholds for behavioral response to heat stimuli applied to the plantar surface of each hindpaw and tail were assessed using a modified Hargreaves test (Hargreaves et al., 1988). All testing was conducted blind with respect to group assignment. Briefly, baseline withdrawal values were calculated from an average of 2 consecutive withdrawal latencies of the tail and the left and the right hindpaws, measured at 15-min intervals. Latencies for the high intensity stimulus at baseline ranged from 2 to 3 s, and a cut-off time of 10 s was imposed to avoid tissue damage. Latencies for the low intensity stimulus at baseline ranged from 6 to 7 s, and a cut-off time of 20 s was imposed to avoid tissue damage. The order of paw and tail testing varied randomly.

**Statistics**

The analgesic responses were calculated as the % of maximal possible effect

$$\text{(\%MPE) using the following equation } \%MPE = \frac{\text{test latency - baseline latency}}{\text{cut off - baseline latency}} \times 100$$

(Carmody, 1995). For high intensity Hargreaves, a cut off of 10 s was used. For low intensity Hargreaves, a 20 s cut off was employed. Since there is no unilateral paw effect expected (or observed) left and right withdrawal latencies were averaged. Statistical significance for analysis of time courses was assessed using a two-way repeated ANOVA with Bonferroni post hoc test and compared the analgesic responses of active treatments versus their vehicle controls. Area under the curves were analyzed with a students t-test. Significance was set at $P < 0.05$. Calculations were conducted with Excel 2003 SP2 (Microsoft, Redmond, CA, USA) & Prism 4.03 (GraphPad, San Diego, CA, USA).

**B. Experiment 1: Ability of ibudilast to induce analgesia alone**

Following baseline withdrawal latency assessments (-45 min), animals received ibudilast or vehicle and were tested again 10 min later to determine if ibudilast or vehicle had any effect on withdrawal latencies. At this time (time 0), the animals received a subcutaneous saline injection (1 ml/kg) to control for conditions in Experiment 2 (see below). Animals were then tested every 10 minutes for 80 min alternating high and low intensity Hargreaves tests.

As shown in Figures 1A-1D, administering ibudilast (7.5 mg/kg intraperitoneally in a dose volume of 2.5 ml/kg in 35% PEG in injection saline) or vehicle (35% PEG in injection saline) had no significant influence on either tail (Figures 1A and 1B) or paw (Figures 1C and 1D) withdrawal latencies in either the high intensity (Figures 1A and 1C) or low intensity (Figures 1B and 1D) Hargreaves test. A subsequent subcutaneous saline injection also produced no significant nociceptive change.

**C. Experiment 2: Ability of ibudilast to potentiate morphine analgesia**

Following baseline withdrawal latency assessments (-45 min), animals received ibudilast or vehicle (-30 min) and were tested again at -10 min. At time 0, animals received a subcutaneous morphine injection (4 mg/kg in a dose volume of 1 ml/kg in injection saline) and were tested every 10 minutes for 230 min alternating high and low intensity Hargreaves tests.

As shown in Figures 2A-2D, administering ibudilast (7.5 mg/kg intraperitoneally in a dose volume of 2.5 ml/kg in 35% PEG in injection saline) 30 min prior to administration of morphine significantly enhanced the pain suppressive effects of morphine on tail (Figures 2A and 2B) and paw (Figures 2C and 2D) withdrawal latencies in high (Figures 2A and 2C) and low (Figures 2B and 2D) intensity Hargreaves tests. Thus, while having little to no effect on its own in the absence of morphine, the glial activation inhibitor ibudilast, when co-administered with morphine, enhanced the efficacy of morphine for control of pain. Analysis of the areas under the curves in Figures 2A-2D confirms the significant increase in analgesia resulting from co-administration of ibudilast with morphine for both the tail (Figure 3A) and hind paw (Figure 3B).

**D. Experiment 3: Ability of ibudilast to potentiate oxycodone analgesia**

Following baseline withdrawal latency assessments (-45 min), animals received ibudilast or vehicle (-30 min) and were tested again at -10 min. At time 0, animals received a subcutaneous oxycodone injection (4 mg/kg in a dose volume of 1 ml/kg in injection saline) and were tested every 10 minutes for 230 min alternating high and low intensity Hargreaves tests.

As shown in Figures 4A-4D, administering ibudilast (7.5 mg/kg intraperitoneally in a dose volume of 2.5 ml/kg in 35% PEG in injection saline) 30 min prior to oxycodone significantly enhanced the pain suppressive effects of oxycodone on tail (Figures 4A and 4B) and paw (Figures 4C and 4D) withdrawal latencies in high (Figures 4A and 4C) and low (Figures 4B and 4D) intensity Hargreaves tests. Thus, while having little to no effect on its own in the absence of oxycodone, ibudilast, when co-administered with oxycodone, enhanced the efficacy of oxycodone for pain control. Analysis of the areas under the curves of Figures 4A-4D confirms the significant increase in analgesia resulting from co-administration of ibudilast with oxycodone for both the tail (Figure 5A) and hind paw (Figure 5B).

EXAMPLE 2

ORAL ADMINISTRATION OF IBUDILAST CONCURRENTLY WITH SUBCUTANEOUS MORPHINE

EXPERIMENTAL PROCEDURES

Male Sprague-Dawley (CD) rats were acclimated to a room temperature of 79 °F to semi-confined conditions on a heated glass surface (79 °F). On the first day, rats were acclimated to a glass surface for 20-30 minutes. Rats were in chambers made of plexiglass and could move freely in either direction. No stimulus was given on day 1. On day 2, rats were acclimated for 5 minutes, and a high intensity beam of light was directed at each rat tail approximately 4 cm from the tip. The time it took for a rat tail to withdraw from the heat source was recorded as the baseline latent period for tail flick. Three trials were done with 5 minutes between trials for each rat. The light source was moved for the 2nd and 3rd trials one cm caudal and rostral of the original trial to avoid tissue damage. Animals were dosed orally with ibudilast (50 mg/kg in vehicle (10% HCO60 + 10% PEG in sterile water) and/or 1.2 mg/kg morphine s.c. formulated in saline for injection. Dose volumes were 1 ml/kg for s.c. administrations (i.e. opioid) and 10 ml/kg for p.o. administrations. Animals were dosed and returned to their cages until 5 minutes before each hourly time point, when they were returned to the glass surface and stimulated with the heat source (3 trials/time point). The ibudilast vehicle was also shown not to have analgesic activity and performed like the saline control. Studies were performed at Avigen Inc. under IACUC regulations.

RESULTS

As shown in Figure 6, morphine (1.2 mg/kg s.c.) and ibudilast (50 mg/kg po) produced weak antinociceptive activity when administered at these doses alone. In contrast, co-administration of oral ibudilast and s.c. morphine results in a substantial, greater-than-additive increase in tail-flick latency times in the combination treatment group versus either individual therapeutic alone. Thus, ibudilast is shown to clearly potentiate morphine-induced analgesia in a classic rat model of analgesia.

Thus, methods of treating acute pain in a subject comprising coadministration of ibudilast with an opioid are disclosed. Ibudilast is used to potentiate the antinociceptive or analgesic efficacy of opioids (*e.g.*, morphine, oxycodone).

Although preferred embodiments of the subject invention have been described in some detail, it is understood that obvious variations can be made without departing from the scope of the invention as claimed herein.

## Claims

1. A phosphodiesterase 4 (PDE-4) inhibitor for use in treating acute pain in a subject undergoing treatment with an opioid.

2. The PDE-4 inhibitor for use according to claim 1, wherein the PDE-4 inhibitor is ibudilast.

3. The PDE-4 inhibitor for use according to any one of claims 1 to 2, wherein the PDE-4 inhibitor or ibudilast is for administration to the subject concurrently with the opioid.

4. The PDE-4 inhibitor for use according to claim 3, wherein the PDE-4 inhibitor, or ibudilast, and the opioid are in the same composition.

5. The PDE-4 inhibitor for use according to either of claims 3 or 4, wherein the PDE4 inhibitor, or ibudilast, and the opioid are in separate compositions.

6. The PDE-4 inhibitor for use according to any one of claims 1 to 5, wherein the opioid is morphine.

7. The PDE-4 inhibitor for use according to any one of claims 1 to 5, wherein the opioid is oxycodone.

8. The PDE-4 inhibitor for use according to any one of claims 1 to 7, wherein the PDE-4 inhibitor is selected from the group consisting of Rolipram, Arofylline, Roflumilast, Pentoxyfylline, and Propentofylline.

9. The PDE-4 inhibitor for use according to any one of the preceding claims, wherein the subject is human.

10. The PDE-4 inhibitor for use according to any one of the preceding claims, wherein the PDE inhibitor, or ibudilast is for systemic administration.

11. The PDE-4 inhibitor for use according to any one of the preceding claims, wherein the PDE inhibitor,or ibudilast is for intravenous, subcutaneous, intraperitoneal, oral, intranasal, or sublingual administration.

12. The PDE-4 inhibitor for use according to any one of the preceding claims, wherein the PDE inhibitor, or ibudilast, and the opioid is for administration with multiple therapeutically effective doses.

## Patentansprüche

1. Phosphodiesterase 4 (PDE-4)-Inhibitor zur Verwendung zum Behandeln von akuten Schmerzen in einem Subjekt, das einer Behandlung mit einem Opioid ausgesetzt ist.

2. PDE-4-Inhibitor zur Verwendung gemäß Anspruch 1, wobei der PDE-4-Inhibitor Ibudilast ist.

3. PDE-4-Inhibitor zur Verwendung gemäß einem der Ansprüche 1 bis 2, wobei der PDE-4-Inhibitor oder Ibudilast zur Verabreichung an das Subjekt zusammen mit dem Opioid ist.

4. PDE-4-Inhibitor zur Verwendung gemäß Anspruch 3, wobei der PDE-4-Inhibitor oder Ibudilast und das Opioid in der gleichen Zusammensetzung vorliegen.

5. PDE-4-Inhibitor zur Verwendung gemäß einem der Ansprüche 3 oder 4, wobei der PDE-4-Inhibitor oder Ibudilast und das Opioid in getrennten Zusammensetzungen vorliegen.

6. PDE-4-Inhibitor zur Verwendung gemäß einem der Ansprüche 1 bis 5, wobei das Opioid Morphin ist.

7. PDE-4-Inhibitor zur Verwendung gemäß einem der Ansprüche 1 bis 5, wobei das Opioid Oxycodon ist.

8. PDE-4-Inhibitor zur Verwendung gemäß einem der Ansprüche 1 bis 7, wobei der PDE-4-Inhibitor ausgewählt wird aus der Gruppe bestehend aus Rolipram, Arofyllin, Roflumilast, Pentoxyfyllin und Propentofyllin.

9. PDE-4-Inhibitor zur Verwendung gemäß einem der vorhergehenden Ansprüche, wobei das Subjekt ein Mensch ist.

10. PDE-4-Inhibitor zur Verwendung gemäß einem der vorhergehenden Ansprüche, wobei der PDE-Inhibitor oder Ibudilast zur systemischen Verabreichung ist.

11. PDE-4-Inhibitor zur Verwendung gemäß einem der vorhergehenden Ansprüche, wobei der PDE-Inhibitor oder Ibudilast zur intravenösen, subkutanen, intraperitonealen, oralen, intranasalen oder sublingualen Verabreichung ist.

12. PDE-4-Inhibitor zur Verwendung gemäß einem der vorhergehenden Ansprüche, wobei der PDE-Inhibitor oder Ibudilast und das Opioid zur Verabreichung in mehreren therapeutisch wirksamen Dosen vorliegt.

## Revendications

1. Inhibiteur de la phosphodiestérase 4 (PDE-4) destiné à une utilisation dans le traitement d'une douleur aiguë chez un sujet en cours de traitement avec un opiacé.

2. Inhibiteur de la PDE-4 destiné à une utilisation selon la revendication 1, dans lequel l'inhibiteur de la PDE-4 est l'ibudilast.

3. Inhibiteur de la PDE-4 destiné à une utilisation selon l'une quelconque des revendications 1 à 2, dans lequel l'inhibiteur de la PDE-4 ou l'ibudilast est destiné à une administration au sujet en même temps que l'opiacé.

4. Inhibiteur de la PDE-4 destiné à une utilisation selon la revendications 3, dans lequel l'inhibiteur de la PDE-4 ou l'ibudilast, et l'opiacé sont dans la même composition.

5. Inhibiteur de la PDE-4 destiné à une utilisation selon l'une ou l'autre des revendications 3 ou 4, dans lequel l'inhibiteur de la PDE-4, ou l'ibudilast, et l'opiacé sont dans des compositions distinctes.

6. Inhibiteur de la PDE-4 destiné à une utilisation selon l'une quelconque des revendications 1 à 5, dans lequel l'opiacé est la morphine.

7. Inhibiteur de la PDE-4 destiné à une utilisation selon l'une quelconque des revendications 1 à 5, dans lequel l'opiacé est l'oxycodone.

8. Inhibiteur de la PDE-4 destiné à une utilisation selon l'une quelconque des revendications 1 à 7, dans lequel l'inhibiteur de la PDE-4 est choisi dans le groupe constitué par le Rolipram, l'Arofylline, le Roflumilast, la Pentoxyfylline, et la Propentofylline.

9. Inhibiteur de la PDE-4 destiné à une utilisation selon l'une quelconque des revendications précédentes, dans lequel le sujet est un humain.

10. Inhibiteur de la PDE-4 destiné à une utilisation selon l'une quelconque des revendications précédentes, dans lequel l'inhibiteur de la PDE, ou l'ibudilast est destiné à une administration systémique.

11. Inhibiteur de la PDE-4 destiné à une utilisation selon l'une quelconque des revendications précédentes, dans lequel l'inhibiteur de la PDE, ou l'ibudilast est destiné à une administration par voie intraveineuse, sous-cutanée, intrapéritonéale, orale, intra-nasale, ou sublinguale.

12. Inhibiteur de la PDE-4 destiné à une utilisation selon l'une quelconque des revendications précédentes, dans lequel l'inhibiteur de la PDE, ou l'ibudilast, et l'opiacé est destiné à une administration avec de multiples doses thérapeutiquement efficaces.

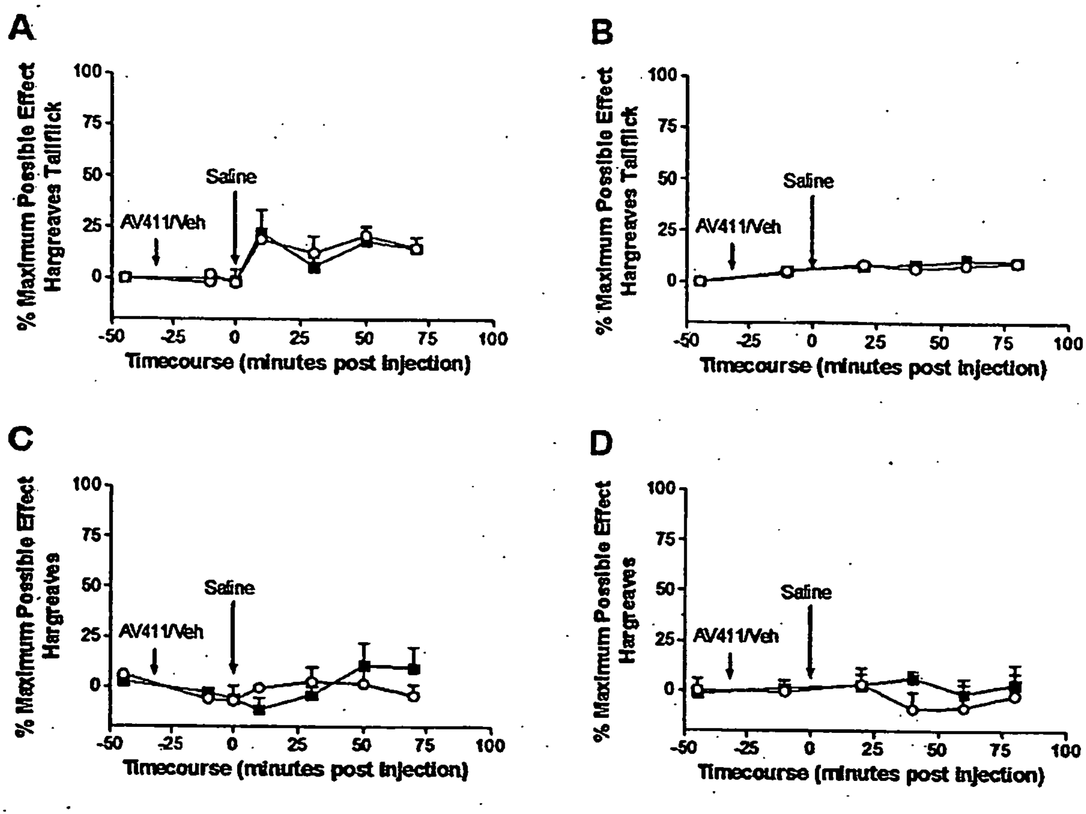
A
% Maximum Possible Effect Hargreaves Tailflick
100
75
50
25
0
Saline
AV411/Veh
-50
-25
0
25
50
75
100
Timecourse (minutes post injection)
B
% Maximum Possible Effect Hargreaves Tailflick
Saline
AV411/Veh
Timecourse (minutes post injection)
C
% Maximum Possible Effect Hargreaves
Saline
AV411/Veh
Timecourse (minutes post injection)
D
% Maximum Possible Effect Hargreaves
Saline
AV411/Veh
Timecourse (minutes post injection)

Figure 1.

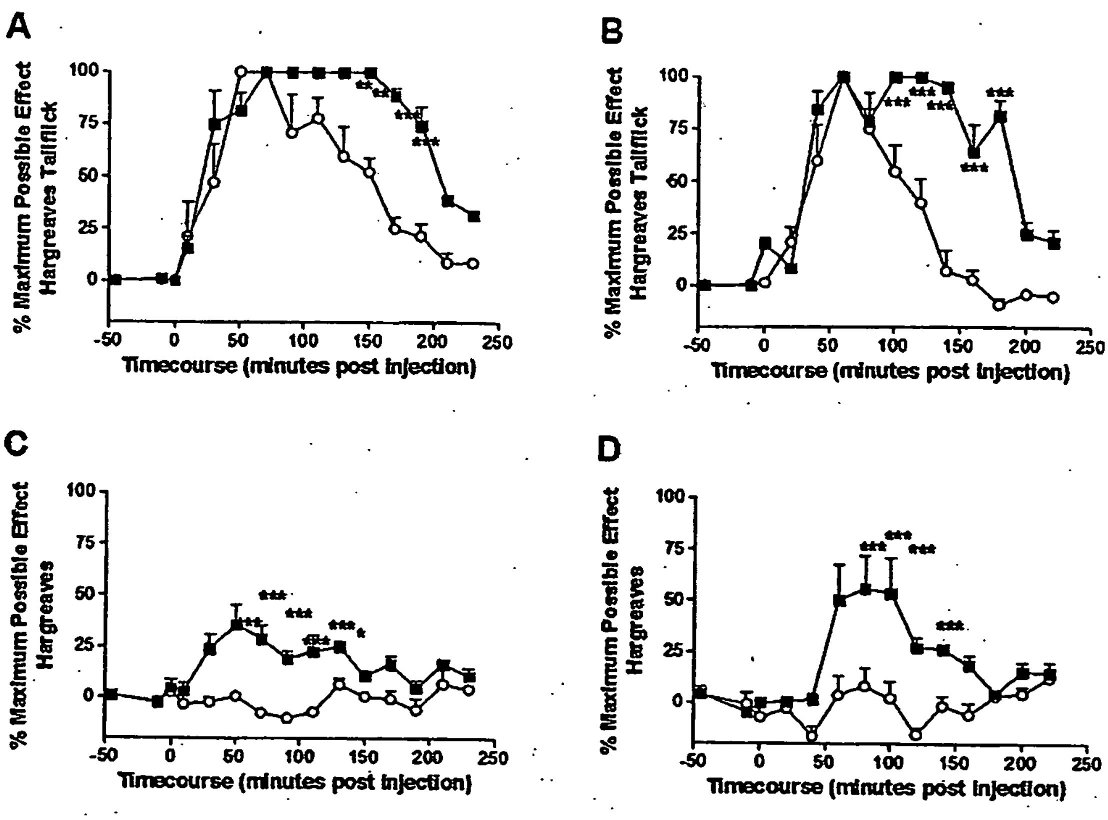
A
% Maximum Possible Effect Hargreaves Tailflick
Timecourse (minutes post injection)
B
% Maximum Possible Effect Hargreaves Tailflick
Timecourse (minutes post injection)
C
% Maximum Possible Effect Hargreaves
Timecourse (minutes post injection)
D
% Maximum Possible Effect Hargreaves
Timecourse (minutes post injection)

Figure 2

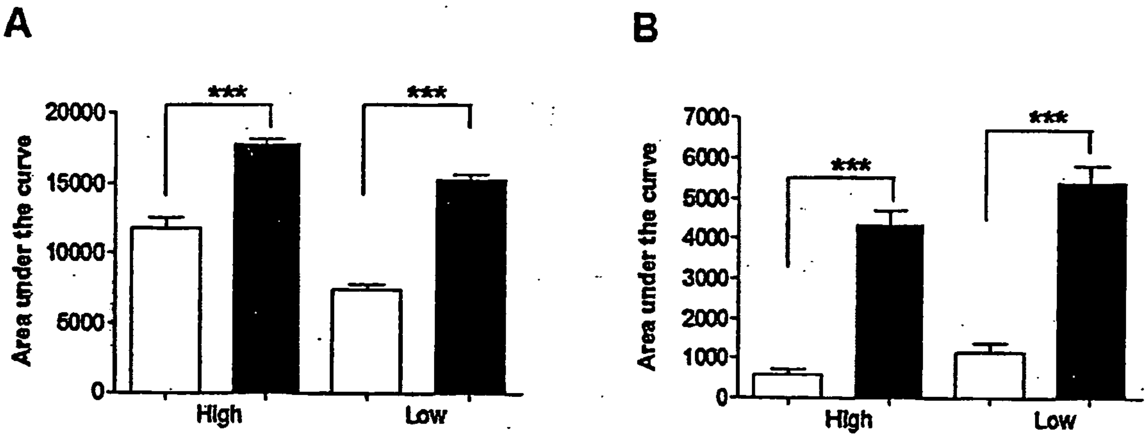
A
B
***
***
***
***
Area under the curve
20000
15000
10000
5000
0
7000
6000
5000
4000
3000
2000
1000
0
High
Low
High
Low

Figure 3

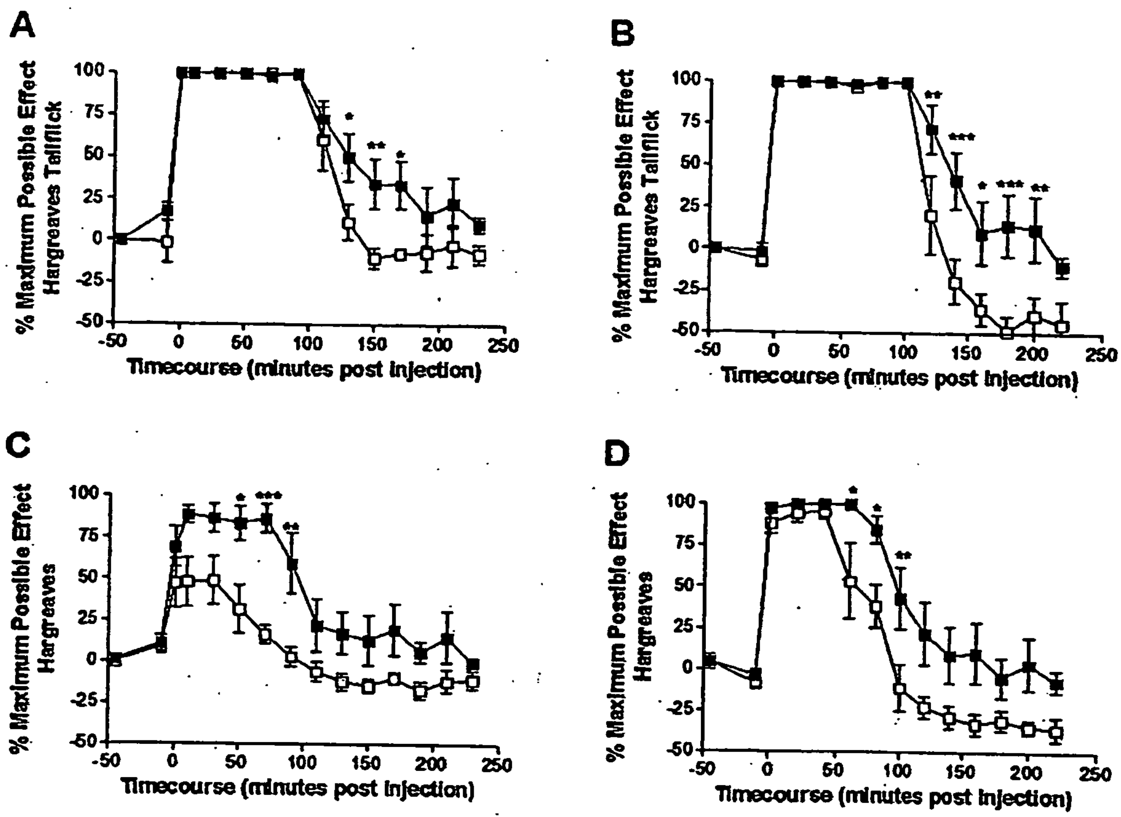
A
B
C
D
% Maximum Possible Effect Hargreaves Tailflick
% Maximum Possible Effect Hargreaves
Timecourse (minutes post injection)
100
75
50
25
0
-25
-50
-50
0
50
100
150
200
250
*
**
***

Figure 4

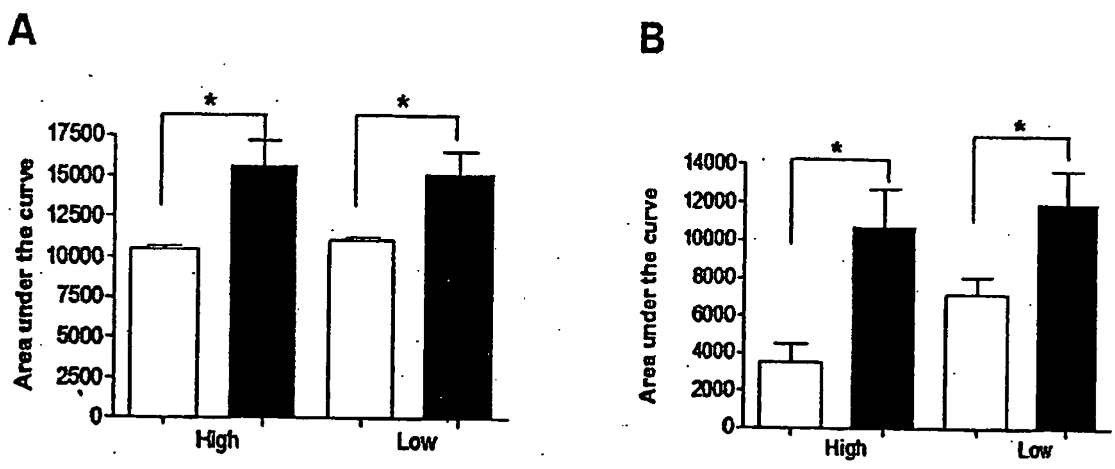
A
*
*
17500
15000
12500
10000
7500
5000
2500
0
Area under the curve
High
Low
B
*
*
14000
12000
10000
8000
6000
4000
2000
0
Area under the curve
High
Low

Figure 5

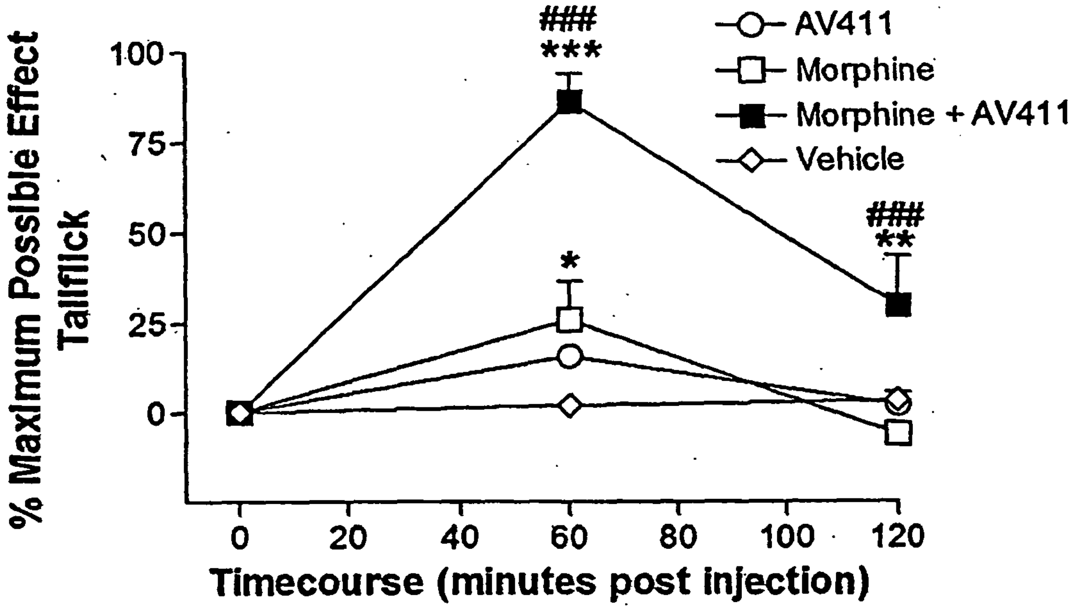

% Maximum Possible Effect Tailflick
100
75
50
25
0
0
20
40
60
80
100
120
Timecourse (minutes post injection)
AV411
Morphine
Morphine + AV411
Vehicle
###
***
*
###
**


Figure 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006063048 A
- WO 2007146087 A
- US 6309634 B


### Non-patent literature cited in the description


- **Sertürner F.** Darstellung der reinen Mohnsäure (Opiumsäure) nebst einer Chemischen Untersuchung des Opiums mir vorzüglicher Hinsicht auf einen darin neu entdeckten Stoff und die dahin gehörigen Bemerkungen. *Journal der Pharmacie fuer Aerzte und Apotheker,* vol. 14 (47-93), 1806
- **Huxtable RJ ; Schwarz SK.** The isolation of morphine-first principles in science and ethics. *Mol Interv.,* October 2001, vol. 1 (4), 189-91
- **Gulland JM ; Robinson R.** *J. Chem. Soc. [London],* 1923, vol. 23 (980
- **Gilman, A.G. ; Goodman, L.S. ; Rall, T.W. ; Murad, F.** The Pharmacological Basis of Therapeutics. MacMillan Publishing Co, 1985
- **WHO.** List of Essential Medicines. March 2005
- **AR Campos et al.** *Biol Pharm Bull,* 2006, vol. 29, 86
- **JA Kiritsy-Roy et al.** *Pharmacol Biochem Behav,* 1989, vol. 32, 717
- **S. Zelcer et al.** *Brain Res,* 2005, vol. 1040, 151
- **Fujimoto, T. et al.** *J. ofNeuroimmunology,* 1999, vol. 95, 35-92
- **Mizuno et al.** *Neuropharmacology,* 2004, vol. 46, 404-411
- News.Medical.Net. *Pharmaceutical News,* 02 August 2005
- **Watkins et al.** *Trends in Neurosciences,* 01 December 2005, vol. 28 (12), 661-669
- **Johnson et al.** *Eur. J of Pain, Saunders, London, GB,* 01 September 2006, vol. 10, S21
- **Ledeboer et al.** *Neuron Glia Biol.,* 2006, vol. 2 (4), 279-291
- **A.L. Lehninger.** Biochemistry. Worth Publishers, Inc,
- **Morrison ; Boyd.** Organic Chemistry. Allyn and Bacon, Inc,
- **J. March.** Advanced Organic Chemistry. McGraw Hill
- Remington: The Science and Practice of Pharmacy
- **J. Griffith Hardman ; L. L. Limbird ; A. Gilman.** Goodman & Gilman The Pharmacological Basis of Therapeutics
- **Gibson.** *Eur. J. Pharmacology,* 2006, vol. 538, 39-42
- Remington: The Science & Practice of Pharmacy. Williams & Williams, 1995
- Physician's Desk Reference. Medical Economics, 1998
- **Kibbe, A.H.** Handbook of Pharmaceutical Excipients. American Pharmaceutical Association, 2000
- **Martin, B.R. et al.** *The Journal of Supportive Oncology,* July 2004, vol. 2
- **Jeffery et al.** *Pharm. Res.,* 1993, vol. 10, 62-368
- **McGee et al.** *J. Microencap.,* 1996
- **Stein et al.** *J. Virol.,* 1999, vol. 73, 3424-3429
- **Davidson et al.** *PNAS,* 2000, vol. 97, 3428-3432
- **Davidson et al.** *Nat.Genet.,* 1993, vol. 3, 219-223
- **Alisky ; Davidson.** *Hum. Gene Ther.,* 2000, vol. 11, 2315-2329
- **Chiang et al.** *Acta Anaesthesiol. Sin.,* 2000, vol. 38, 31-36
- **Jain, K.K.** *Expert Opin. Investig. Drugs,* 2000, vol. 9, 2403-2410
- **D'Amour et al.** *J. Pharmacol. Exp. and Ther.,* 1941, vol. 72, 74-79
- **Randall et al.** *Arch. Int. Pharmacodyn.,* 1957, vol. 111, 409-419
- **Vinegar et al.** *J. Phamacol. Exp. Ther.,* 1969, vol. 166, 96-103
- **Dubuisson et al.** *Pain,* vol. 4, 161-174
- **Kim et al.** *Pain,* 1992, vol. 50, 355-363
- **Chaplan et al.** *J. Neurosci. Methods,* 1994, vol. 53, 55-63
- **Bennett et al.** *Pain,* 1988, vol. 33, 87-107
- **Gogas et al.** *Analgesia,* 1997, vol. 3, 111-118
- **SH Kim ; JM Chung.** *Pain,* 1992, vol. 50, 355-363
- **Hargreaves et al.** *Pain,* 1998, vol. 32, 77-88